Europäisches Patentamt

European Patent Office

Office européen des brevets

(11) Publication number: **0 411 794 A1**

# EUROPEAN PATENT APPLICATION

(21) Application number: **90307967.1**

(22) Date of filing: **20.07.90**

(51) Int. Cl.5: **C12M 1/00, B25J 21/02, G21F 7/04**

(30) Priority: **20.07.89 GB 8916578**

(43) Date of publication of application:
**06.02.91 Bulletin 91/06**

(84) Designated Contracting States:
**AT BE CH DE DK ES FR GB GR IT LI LU NL SE**

(71) Applicant: **Foster, William Joseph**
**Elektrotek Elektrical Eng. Ltd., Unit 32, Mantra House, South Street**
**Keighley, BD21 1SC(GB)**

(72) Inventor: **Foster, William Joseph**
**Elektrotek Elektrical Eng. Ltd., Unit 32, Mantra House, South Street**
**Keighley, BD21 1SC(GB)**

(74) Representative: **Browne, Robin Forsythe**
**Urquhart-Dykes & Lord Tower House Merrion WayWay**
**Leeds LS2 8PA West Yorkshire(GB)**

(54) **Anaerobic cabinet.**

(57) An anaerobic cabinet for manipulation of air sensitive compounds includes inlets adapted to mix separate supplies of nitrogen. carbon dioxide and a mixture of nitrogen, carbon dioxide and hydrogen.

EP 0 411 794 A1

## ANAEROBIC CABINET

This invention relates to anaerobic cabinets. Anaerobic cabinets find application in culture of anaerobic micro-organisms and the manipulation of oxygen sensitive compounds. Such cabinets incorporate airlocks through which articles may be placed into the cabinets. In addition a bare hand working facility may be provided.

Conventional cabinets are supplied with a readymade gaseous mixture of 10% hydrogen. 10% carbon dioxide and 80% nitrogen. The hydrogen is provided to remove oxygen from the cabinet. a catalyst. eg palladium. being disposed within the cabinet for this purpose. A gaseous mixture in the above ratio is safe from explosion but is expensive. Alternative arrangements wherein much cheaper supplies of pure gases are mixed within the cabinet are extremely dangerous on account of the explosive nature of pure hydrogen. Flame proofing of such cabinets is very expensive.

According to a first aspect of the present invention an anaerobic cabinet includes inlets adapted to mix separate supplies of nitrogen carbon dioxide and a mixture of nitrogen carbon dioxide and hydrogen.

The inlet may include valves and pressure flow regulators. A cabinet may also include a detector arranged to provide a signal indicative of a supply of any of the gases falling below a predetermined minimum pressure.

Dilution of the hydrogen containing mixed gas allows economic use of the latter. A total proportion of hydrogen within the cabinet of approximately 1 to 3% preferably 2.5% is adequate to ensure anaerobic conditions. Use of a cabinet in accordance with the first aspect of the present invention allows the proportions of nitrogen and carbon dioxide to be selected in accordance with a user's precise requirements. Greater proportions of hydrogen may be employed if required. Avoidance of use of pure hydrogen allows conventional electrical circuitry to be employed without hazard.

Bare arm working facilities of prior art anaerobic cabinets have not been satisfactory. These generally incorporate an aperture in the wall of the cabinet closable by means of a bung assembly, and a rubber sleeve extending outwardly from the aperture and arranged to engage a user s arm. Means for evacuating and flushing the interior of the rubber sleeve prior to opening the bung assembly is complicated and requires use of a vacuum pump. When a user's hands need to be inserted into the cabinet, the hands are placed inside the sleeves and the latter sealed to the user's arms. The evacuation valve is opened and the pump switched on to remove air from the sleeve surrounding the user's hands and arms. The pump is then stopped, the evacuation valve closed and anaerobic gas admitted. The sealing bung may then be released allowing the user access to the interior of the cabinet. Manipulation of the bung into and out of the aperture in the wall of the cabinet is often difficult.

According to a second aspect of the present invention an anaerobic cabinet includes an aperture in the wall of the cabinet a flexible sleeve extending outwardly from the aperture, the sleeve terminating in a non-deformable collar having an opening into which a user's arm may be inserted, sealing means adapted to form an airtight seal between the collar and a user's arm and a removable stopper arranged to seal the opening when not in use.

The bare hand facility in accordance with the second aspect of this invention affords a simple and easily used arrangement. It does not require any solenoid valves, vacuum pumps. piping or a sophisticated bung assembly. The arrangement may rely on the positive pressure within the main chamber of the anaerobic cabinet to exclude oxygen from the cabinet when the seals are opened. The opening in the sleeve is small facilitating rapid egress of gas from the cabinet, preventing ingress of oxygen. The use of a non-deformable collar minimises suction of oxygen into the cabinet by minimising turbulence from the end of the sleeve.

The arrangement confers several advantages. The stopper may be arranged to be heavy enough so that the sleeve depends from the front of the cabinet so that the latter takes up minimal laboratory space. The stopper. being inserted from the interior of the collar is held in position by gravity. preventing accidental dislodgement from the collar. The stopper may be significantly smaller than that required by a prior art arrangement. facilitating manipulation by a user and minimising the obstruction caused by it to working within the cabinet.

According to a third aspect of the present invention an anaerobic cabinet includes an aperture closable by a door and a sealing arrangement adapted to prevent passage of air when the door is closed, the sealing arrangement including an inflatable member surrounding the perimeter of the aperture and arranged to engage the door and cabinet when inflated forming an airtight seal.

In preferred embodiments the door is slidable or closable by a latch and the inflatable member is arranged to bear against the door and wall of the cabinet with a force sufficient to lock and prevent accidental opening of the door.

The inflatable member may be arranged to be inflated by pressurised gas for example the gas cylinder used to supply nitrogen to the cabinet.

This arrangement is superior to the conventional use of an O ring around the perimeter of the door, to which a substantial force is applied to create the seal. In such arrangements the force may be generated by a mechanical cam arrangement or by means of a screw mechanism acting on a fulcrum. The arrangement of the present invention has the further advantage that the door may be easily opened by one hand, even when the user is using a glove or gauntlet. Moreover the sealing and locking arrangement may be fully automated.

Preferred embodiments of of the present invention incorporate an airlock containing gas at a pressure greater than atmospheric pressure.

This arrangement allows the airlock to be easily flushed without use of a vacuum pump. A palladium catalyst may be disposed within the airlock to remove any unwanted oxygen.

The invention is further described by means of example but not in any limitative sense. with reference to the accompanying drawings of which:

Figure 1 shows a cabinet in accordance with the invention.

Figure 2 is an end elevation of the cabinet shown in Figure 1,

Figure 3 is a schematic view of the gas supply to the cabinet.

Figure 4 is a cross section of the bare arm facility,

Figure 5 illustrates gas supply to the door seal,

Figure 6 shows a door of a cabinet in accordance with this invention,

Figure 7 is a cross section on AA of Figure 6,

Figure 8 is an inner door in accordance with this invention, and

Figure 9 is a cross section of BB of Figure 8.

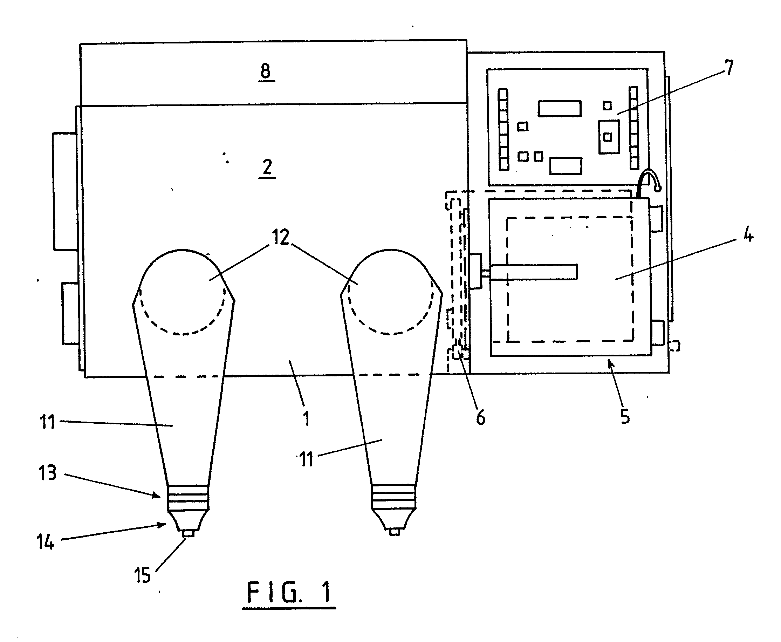

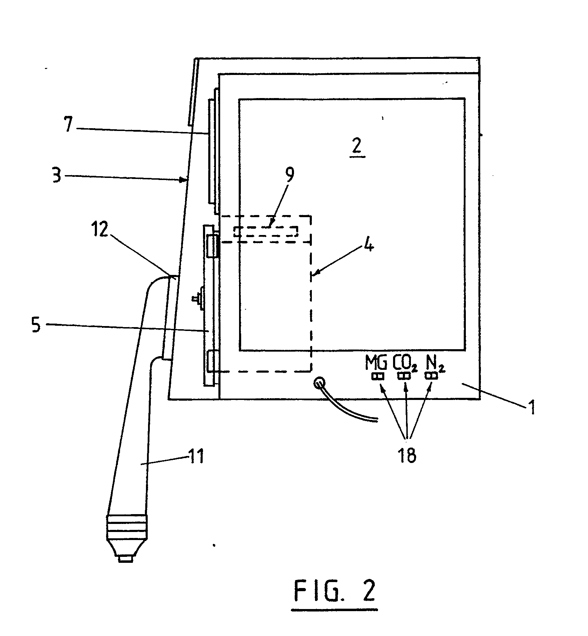

The cabinet shown in Figures 1 and 2 comprises a casing 1 having a main chamber 2 and a clear acrylic front window 3. An airlock 4 has a hinged outer door 5 and sliding inner door 6. A control panel 7 carries instrumentation for operation of the anaerobic cabinet. An area 8 of the main chamber incorporates gas circulating fans and oxygen removing catalysts. A deoxygenation catalyst 9 is disposed within the airlock 4. A condensor 10 serves to remove water vapour from the atmosphere within the cabinet.

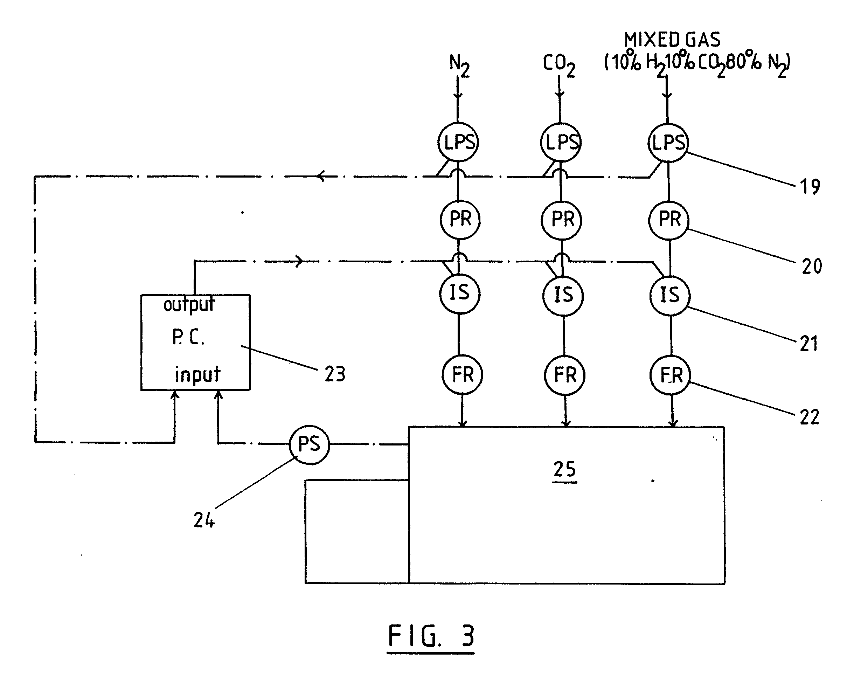

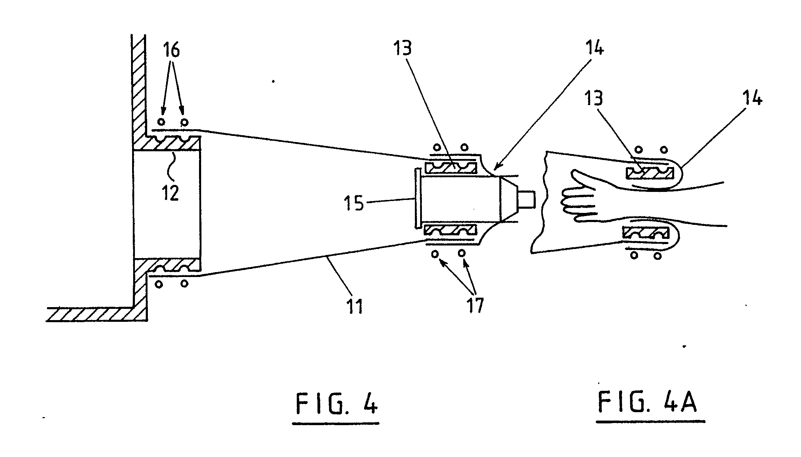

A pair of flexible plastic sleeves 11 depend from apertures 12 in the front face of the cabinet. The sleeves taper from the apertures 12. The ends of the sleeves remote from the apertures are terminated by rigid cuffs 13 which may be sealed by bungs or stoppers 15. Flexible sleeves 14 serve to seal the rigid collar 13 to a user's arm as shown in Figure 4a. O rings 16, 17 seal ends of the sleeve 11 to the aperture 12 and collar 13 respectively. The gas supply to the cabinet is illustrated in Figure 3. Inlets 18 (see Figure 2) are adapted for connection to cylinders containing nitrogen. $CO_2$. and mixed gas containing 10% $H_2$ 10% $CO_2$ and 80% $N_2$. Low pressure sensors 19 are arranged to generate an alarm in the event that the pressure within the respective cylinder falls below a predetermined minimum value. Pressure regulators 20 serve to regulate the supply to inlet solenoid valves 21 and flow regulators 22. An electronic programable control23 serves to control action of the solenoids 21 in response to signals from a pressure sensor 24 communicating with the main chamber 25. The supplies of gases to the main chamber are arranged so that the concentration of hydrogen therein lies between 1 and 3%, preferbly 2.5%. This concentration is sufficient to remove any traces of oxygen from within the cabinet by combination of the latter in the presence of the catalysts disposed in the compartment 8. Greater concentrations of hydrogen may be used if required.

In use of the bare hand facility a user inserts his hand through the rigid collar 13 pushing the bung 15 inwardly. The positive gas pressure within the cabinet and small dimension of the aperture of the collar 13 provides a sufficiently strong outward flow of gas to prevent ingress of oxygen. The smooth aperture formed by the mouth of the flexible sleeve 14 minimises turbulence at the end of the glove. The small dimension of the stoppers facilitates manipulation of the latter through the apertures 12 during opening and closing of the bare hand facility.

When not in use the flexible sleeves, collars and stoppers hang from the face of the cabinet minimising obstruction to personnel and causing the stoppers to be retained under the action of gravity.

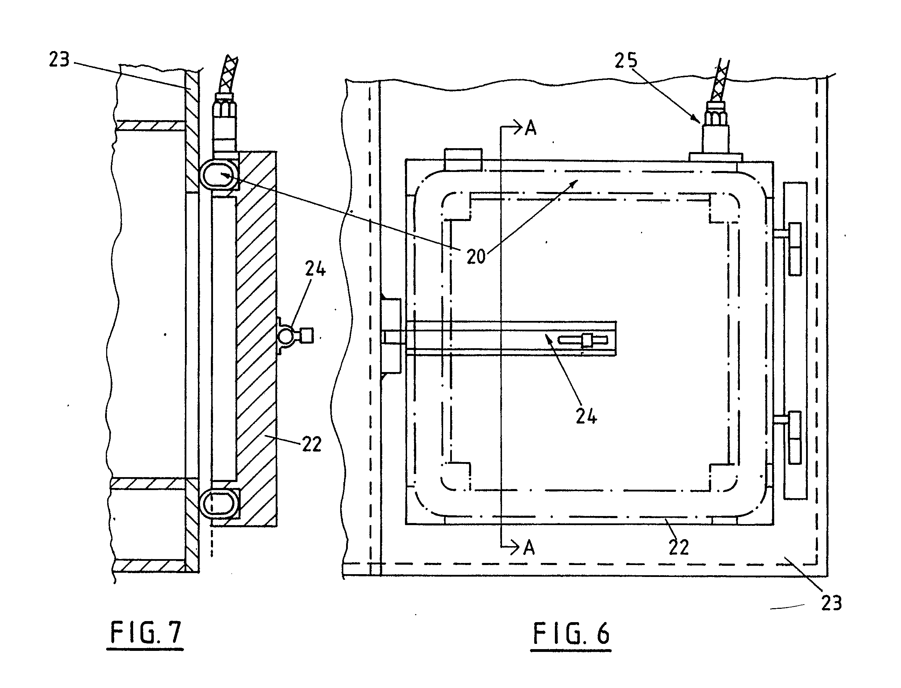

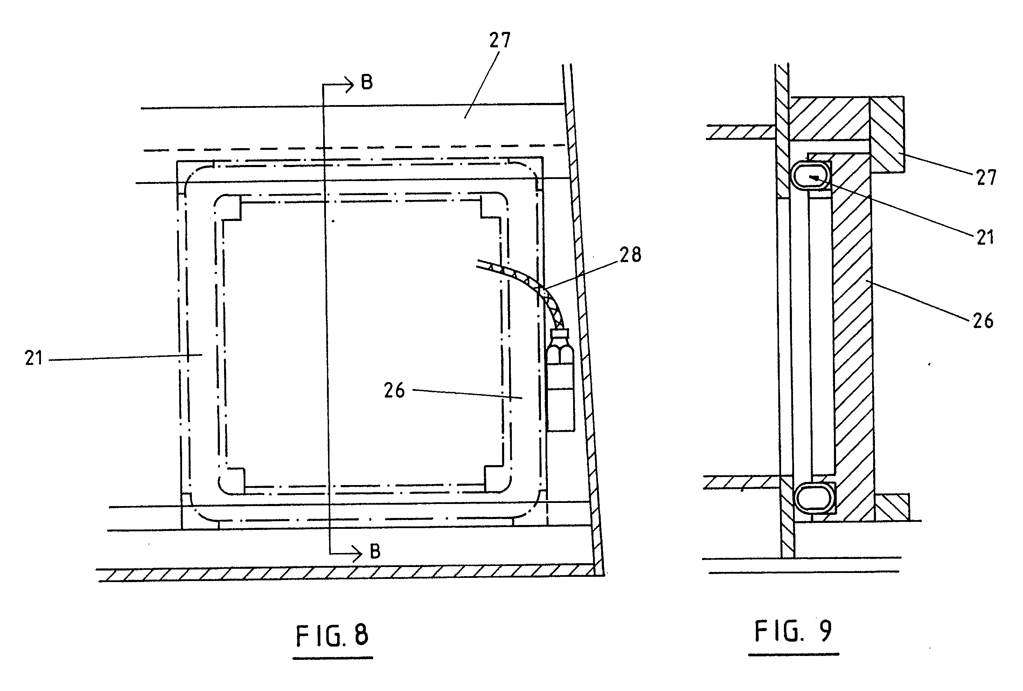

The construction of the airlock doors is shown in Figures 6 to 9.

Anaerobic cabinets require a locking system built onto the main chamber of the cabinet so that culture dishes and the like can be placed inside and removed from the main chamber without losing the anaerobic conditions. The lock system requires two doors, one to the outside. called the outer door, and the other from the lock to the main chamber, called the inner door. These doors require seals to prevent the escape of the anaerobic gas mixture and to prevent oxygen entering the main chamber. It is an advantage to have the doors locked so that they cannot be opened simultaneously. Conventional methods of locking doors of anaerobic cabinets include use of "O" rings surrounding the doors. seals being created by application of great mechanical force to the O rings. Previously known systems suffer from the disadvantage that the inner door is difficult to open with one hand, particularly when the user is wearing gloves or is using the bare arm facility. The present invention provides a fully automatic system. The O rings are replaced by inflatable tubes 20, 21 disposed in the outer and inner doors respectively. The outer door 22 is secured to the frame 23 by means of a sliding bolt 24. Inflation of the ring 20 by compressed nitrogen delivered from the gas supply by means of a hose 25 causes the ring 20 to bear against the door and frame creating a seal between the latter. The outward pressure acting on the bolt 24 prevents accidental opening of the latter. A switch (not shown) is arranged to sense closure of the bolt 24 to provide a signal indicative that the door is locked to the control arrangement. Thus the door is secure when the bolt is closed and the gas supply connected to the ring 20.

The inner door operates on a similar principle, the door 26 being arranged to slide in a runner 27. A gas supply 28 is connected to the inflatable ring 21 and causes the latter to seal and secure the door when inflated, preventing accidental opening of the latter.

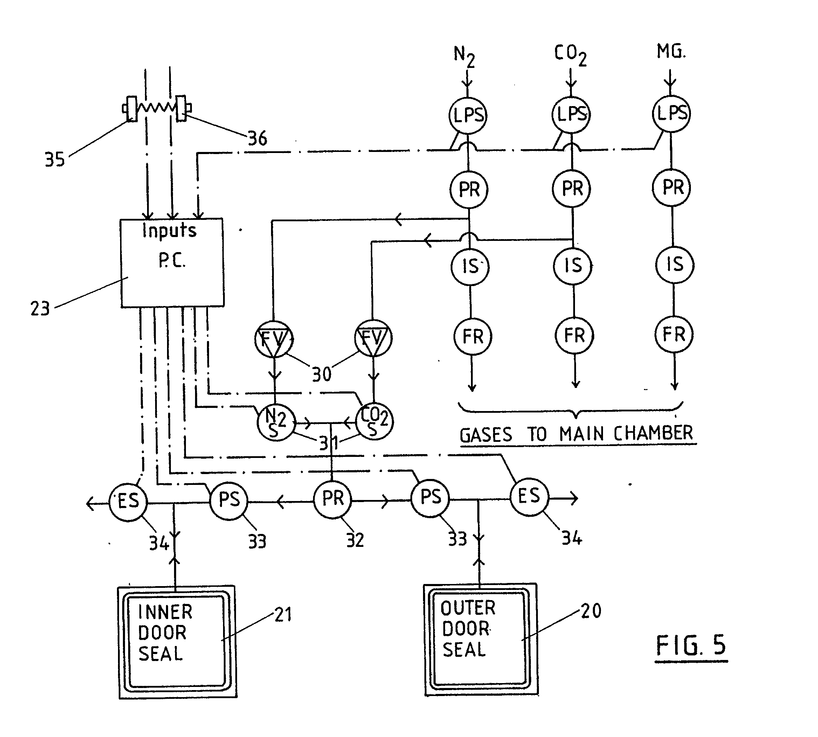

The control of the doors is illustrated in Figure 5, the gas supply to the main chamber being shown in a similar manner to Figure 3. One way flow valves 30 provide supplies of nitrogen and carbon dioxide to solenoids 31 and a pressure regulator 32. Sealed pressurisation solenoids 33 are arranged to deliver supplies of gas to the inner and outer inflatable door seals 21, 20. Exhaust solenoids 33, 34 allow discharge of the inflatable seals 20, 21 to the atmosphere. Operation of the solenoids is controlled by the programable controller 23 actuated by means of switches 35. 36.

The pressure within the main chamber is arranged to excede atmospheric pressure, gas from the main chamber being fed via a solenoid to the airlock. Maintenance of the airlock at a pressure greater than atmospheric pressure prevents ingress of oxygen without need for provision of a vacuum pump.

## Claims

1. An anaerobic cabinet characterised by including inlets adapted to mix separate supplies of nitrogen, carbon dioxide and a mixture of nitrogen, carbon dioxide and hydrogen.
2. An anaerobic cabinet as claimed in claim 1 characterised in that the inlets include low pressure sensors arranged to actuate an alarm if pressure within a respective gas supply falls below a minimum value.
3. An anaerobic cabinet as claimed in claim 1 including an aperture in the wall of the cabinet, a flexible sleeve extending outwardly from the aperture, characterised in that the sleeve terminates in a non-deformable collar having an opening into which a user's arm may be inserted. sealing means being adapted to form an airtight seal in use between the collar and the user's arm and a removable stopper arranged to seal the opening when the sleeve is not in use.
4. A cabinet as claimed in claim 3 characterised in that the stopper is inserted into the opening from the interior of the collar.
5. A cabinet as claimed in any preceding claim including an aperture closable by a door and a sealing arrangement adapted to prevent passage of air when the door is closed, characterised in that the sealing element includes an inflatable member surrounding the perimeter of the aperture and arranged to engage the door and cabinet when inflated to form an airtight seal.
6. An anaerobic cabinet as claimed in claim 5 wherein said door is slidable or closable by a latch and characterised in that the inflatable member is arranged to bear against the door and wall of the cabinet with a force sufficient to lock and prevent accidental opening of the door.
7. An anaerobic cabinet as claimed in claim 6 characterised in that said inflatable member is arranged to be inflated by pressurised gas.
8. An anaerobic cabinet as claimed in any preceding claim characterised in including an air lock containing gas of a pressure greater than atmospheric pressure.
9. An anaerobic cabinet including an aperture in the wall of the cabinet and a flexible sleeve extending outwardly from the aperture, characterised in that the sleeve terminates in a non-deformable collar having an opening into which a user's arm may be inserted and sealing means adapted to form an air-tight seal between the collar an a user's arm and a removable stopper arranged to seal the opening when not in use.
10. An anaerobic cabinet as claimed in claim 9 wherein the stopper is adapted to be inserted into the opening from the interior of the collar.
11. An anaerobic cabinet including an aperture closable by a door and a sealing arrangement adapted to prevent passage of air when the door is closed. characterised in that the sealing arrangement includes an inflatable member surrounding the perimeter of the aperture and arranged to engage the door and cabinet when inflated to form an airtight seal.
12. An anaerobic cabinet as claimed in claim 11 wherein the door is slidable or closable by a latch and characterised in that the inflatable member is arranged to bear against the door and wall of the cabinet with a force sufficient to lock and prevent accidental opening of the door.
13. An anaerobic cabinet as claimed in claim 12 characterised in that the inflatable member is arranged to be inflated by pressurised gas from the nitrogen cylinder.

14. An anaerobic cabinet as claimed in claim 13 characterised in including an air lock containing gas at a pressure greater than atmospheric pressure.

FIG. 1

FIG. 2

N₂  CO₂  MIXED GAS
(10% H₂ 10% CO₂ 80% N₂)

FIG. 3

EP 0 411 794 A1

FIG. 4A

FIG. 4

FIG. 5

EP 0 411 794 A1

FIG. 7

FIG. 6

EP 0 411 794 A1

FIG. 9

FIG. 8

European
Patent Office

**EUROPEAN SEARCH
REPORT**

Application Number

**EP 90 30 7967**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 168 915   (DON WHITLEY SCIENTIFIC LTD) <br> * Figures 1,2; claims * <br> – – – | 1 | C 12 M 1/00 <br> B 25 J 21/02 <br> G 21 F 7/04 |
| A | GB-A-2 174 714   (DON WHITLEY SCIENTIFIC LTD) <br> – – – | | |
| A | FR-A-2 254 409   (M.E. COX et al.) <br> * Figure 6 * <br> – – – | 3,9 | |
| A | FR-A-2 310 095   (A. PUJOL) <br> * Figures 1,2 * <br> – – – – – | 5-7 | |

| | TECHNICAL FIELDS SEARCHED (Int. Cl.5) |
|---|---|
| | C 12 M <br> B 01 L <br> F 25 J <br> G 21 F |

The present search report has been drawn up for all claims

| Place of search | Date of completion of search | Examiner |
|---|---|---|
| The Hague | 21 November 90 | COUCKE A.O.M. |